(19) Europäisches Patentamt
European Patent Office
Office européen des brevets

(11) **EP 3 717 695 B1**

(12) **EUROPÄISCHE PATENTSCHRIFT**

(45) Veröffentlichungstag und Bekanntmachung des Hinweises auf die Patenterteilung:
**07.07.2021 Patentblatt 2021/27**

(21) Anmeldenummer: **18807594.9**

(22) Anmeldetag: **20.11.2018**

(51) Int Cl.:
*D21F 5/18* *(2006.01)*      *D21F 11/00* *(2006.01)*
*D21F 11/14* *(2006.01)*      *D21G 9/00* *(2006.01)*

(86) Internationale Anmeldenummer:
**PCT/EP2018/081831**

(87) Internationale Veröffentlichungsnummer:
**WO 2019/101701 (31.05.2019 Gazette 2019/22)**

(54) **VERFAHREN ZUR BESTIMMUNG DES TROCKENGEHALTS EINER FASERSTOFFBAHN UND VERFAHREN ZUM STEUERN ODER REGELN EINER MASCHINE ZUR HERSTELLUNG EINER PAPIERBAHN, SOWIE COMPUTERPROGRAMM ZUR DURCHFÜHRUNG DER VERFAHREN**

METHOD FOR DETERMINING THE DRYNESS OF A FIBROUS WEB, AND METHOD FOR CONTROLLING OR REGULATING A MACHINE FOR PRODUCING A PAPER WEB, AND COMPUTER PROGRAM FOR CARRYING OUT THE METHODS

PROCÉDÉ DE DÉTERMINATION DE LA TENEUR EN MATIÈRE SÈCHE D'UNE BANDE DE MATIÈRE FIBREUSE ET PROCÉDÉ DE COMMANDE OU DE RÉGULATION D'UNE MACHINE DE PRODUCTION D'UNE BANDE DE PAPIER, AINSI QUE PROGRAMME INFORMATIQUE PERMETTANT LA MISE EN OEUVRE DES PROCÉDÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.11.2017 DE 102017127932**

(43) Veröffentlichungstag der Anmeldung:
**07.10.2020 Patentblatt 2020/41**

(73) Patentinhaber: **Voith Patent GmbH**
**89522 Heidenheim (DE)**

(72) Erfinder:
• **ACHTERMANN, Jan**
**88263 Horgenzell (DE)**

• **SCHWIER, Marcus**
**88074 Meckenbeuren-Brochenzell (DE)**
• **POPP, Marco**
**96260 Weismain (DE)**
• **SCHÄFER, Jürgen**
**88212 Ravensburg (DE)**

(74) Vertreter: **Voith Patent GmbH - Patentabteilung**
**St. Pöltener Straße 43**
**89522 Heidenheim (DE)**

(56) Entgegenhaltungen:
**US-A1- 2004 118 009      US-B1- 9 284 686**

EP 3 717 695 B1

**Beschreibung**

Die Erfindung umfasst ein Verfahren zur Steuerung und/oder Regelung einer Maschine zur Herstellung einer Faserstoffbahn, insbesondere einer Tissuebahn gemäß dem Oberbegriff des Anspruchs 4, sowie ein Verfahren zu Bestimmung des Trockengehalts einer Faserstoffbahn gemäß dem Oberbegriff des Anspruchs 1.

Die Erfindung betrifft weiter ein Computerprogramm zur Durchführung eines solchen Verfahrens sowie ein Computerprogrammprodukt mit einem derartigen Computerprogramm.

Ein zentrales Elemente bei der Herstellung von Tissuebahnen - aber auch anderer Faserstoffbahnen- ist ein sogenannter Yankeezylinder. Dabei handelt es sich um einen meist dampfbeheizten Zylinder mit vergleichsweise großem Durchmesser, über den die Faserstoffbahn zum Trocknen geführt wird. Zusätzlich ist einem solchen Zylinder noch mindestens eine -häufig zwei-Trockenhaube zugeordnet. Mittels einer oder mehreren Trockenhauben wird durch Gas und/oder Dampf aufgeheizte Luft an die Oberfläche der Faserstoffbahn gebracht, um die Trocknung der Bahn weiter zu beschleunigen. Eine solche Vorrichtung ist beispielsweise in der DE 10 2012 203 035 beschrieben.

Das Dokument US 9 284 686 B1 beschreibt ein Verfahren zum Steuern und Regeln einer Papiermaschine.

Um einen optimalen Betrieb der Maschine zu ermöglichen, muss die Temperatur dieser heißen Luft auf die Beschaffenheit der Faserstoffbahn, abgestimmt sein. Hierfür ist die Kenntnis des Trockengehalts dieser Faserstoffbahn vor den Yankeezylinder eine wichtige Kenngröße.

Die Bestimmung des Trockengehalts einer Faserstoffbahn ist generell aufwändig. Üblicherweise ist dazu eine Probennahme notwendig. Von dieser Probe wird im Labor dann der Trockengehalt der Bahn bestimmt. In der Regel dauert es mehrere Stunden um die Proben vollständig zu trocknen und den Trockengehalt zu bestimmen. Eine Steuerung, und insbesondere eine Regelung unter Verwendung eines solchen Messwertes sind kaum möglich.

Zudem ist die Probennahme direkt vor dem Yankee sehr schwierig, da die Gefahr gesteht, dass entweder die Oberfläche des Yankeezylinders und /oder ein Filz einer dort häufig angeordneten Presseneinheit bei der Probennahme beschädigt werden. Zudem ist die Probennahme an diesen Positionen auch für das Personal nicht ungefährlich, weshalb sie an vielen Maschinen aus Sicherheitsgründen gar nicht erlaubt ist.

Es ist eine Aufgabe der Erfindung, die Möglichkeit zur Steuerung oder Regelung einer Maschine zur Herstellung einer Faserstoffbahn, insbesondere einer Tissuemaschine zu verbessern.

Es ist eine weitere Aufgabe der Erfindung, die Sicherheit für das Betreiberpersonal speziell hinsichtlich der Probennahme gegenüber dem Stand der Technik zu erhöhen. Es ist einer weitere Aufgabe der Erfindung, eine Steuerung oder Regelung vorzuschlagen, die kostengünstig und ohne großen Aufwand realisierbar ist.

Die Aufgaben werden vollständig gelöst durch ein Verfahren zur Bestimmung des Trockengehalts einer Faserstoffbahn gemäß Anspruch 1, ein Verfahren zum Steuern oder Regeln einer Maschine zur Herstellung einer Faserstoffbahn gemäß Anspruch 4, ein Computerprogramm gemäß Anspruch 9 sowie ein Computerprogrammprodukt gemäß Anspruch 10.

Hinsichtlich der Bestimmung des Trockengehalts einer Faserstoffbahn wird die Aufgabe gelöst durch ein Verfahren zur Bestimmung des Trockengehalts einer Faserstoffbahn, insbesondere einer Tissuebahn, während der Herstellung der Faserstoffbahn in einer Maschine, wobei die Maschine einen Trockenzylinder, insbesondere einen Yankeezylinder umfasst, dem zumindest eine, vorzugsweise zwei Trockenhauben, zugeordnet sind, sowie eine Aufrollung zum Aufwickeln der Faserstoffbahn dadurch gekennzeichnet, dass die Bestimmung des Trockengehalts der Faserstoffbahn vor dem Trockenzylinder auf Basis von Messwerten erfolgt, die die folgenden Größen Beschreiben:

a) Feststoffmenge an der Aufrollung
b) Wassermenge an der Aufrollung
c) Wassermenge, die in der oder den Trockenhauben verdampft wird.

Vorteilhafte Ausführungen werden in den Unteransprüchen beschrieben.

Üblicherweise werden die Fasermengen und die Wassermengen für die Größen nach a) b) und c) in kg/h oder in t/h gemessen bzw. angegeben. Es kann aber auch vorgesehen sein, dass die Größen für a), b) c) in anderer Form vorliegen.

Wenn im Rahmen dieser Anmeldung davon gesprochen wird, dass der Trockengehalt der Faserstoffbahn ermittelt wird, ist dem Fachmann auf dem Gebiet klar, dass stets auch alternativ der Feuchtegehalt der Faserstoffbahn ermittelt werden kann. Diese beiden Werte, die sich stets zu 100% addieren, beschreiben dabei denselben Sachverhalt.

Häufig wird die Lufttemperatur in Trockenhauben, die für Aspekte dieser Erfindung geeignet sind, vorteilhafterweise zwischen 150°C und 500° C, insbesondere zwischen 250°C und 400°C liegen. Bei dampfbeheizten Trockenhauben überschreitet die Temperatur selten 200°C. Für höhere Temperaturen kommen üblicherweise gasbeheizte Trockenhauben zum Einsatz.

Vorteilhafterweise kann die Bestimmung des Trockengehalts der Faserstoffbahn vor dem Trockenzylinder dadurch erfolgen, dass aus der Wassermenge an der Aufrollung und der Wassermenge, die in der Trockenhaube verdampft wird, die Wassermenge ermittelt wird, die vor dem Trockenzylinder in der Faserstoffbahn vorhanden war.

Da nach dem Trockenzylinder, insbesondere dem Yankeezylinder oft keine nennenswerte Trocknung der Faserstoff bahn mehr geschieht, kann dies beispielsweise durch einfache Addition geschehen. Wird diese ermittelte Wassermenge zu der Gesamtmasse der Bahn in Relation gesetzt, so ergibt sich der entsprechende Trockengehalt.

Eine mögliche Realisierung der Trockengehaltsbestimmung kann also wie folgt aussehen:

$$TG = \frac{Feststoff\,[\frac{kg}{h}]}{H2O\,(am\,Roller)\,[\frac{kg}{h}] + H2O\,(verdampft)\,[\frac{kg}{h}] + Feststoff\,[\frac{kg}{h}]}$$

Es können aber auch Abwandlungen dieser Bilanzrechnung verwendet werden. So kann beispielsweise durch Anpassung der Formal der Tatsache Rechnung getragen werden, dass sich die Feuchte der Faserstoffbahn zwischen dem Ablösen vom Trockenzylinder und der Aufrollung noch ändern kann. Dabei kann der Trockengehalt der Bahn sich auf dieser Strecke prinzipiell erhöhen oder auch erniedrigen. Dies kann beispielsweise durch einen empirisch bestimmten Gewichtungsfaktor geschehen.

In weiteren vorteilhaften Ausführungen kann vorgesehen sein, dass die Maschine Messvorrichtungen umfasst, um zumindest eine, vorzugsweise alle Größen zu a) b) und/oder c) während des laufenden Betriebs der Maschine online zu erfassen.

In vielen Anwendungen wird insbesondere die Größe c), also die Menge des in der Haube oder den Hauben verdampften Wassers online bestimmt werden. Dies kann beispielsweise durch Messungen an der Haubenabluft erfolgen. Über den Abluftstrom können beispielsweise kontinuierlich oder in regelmäßigen Abständen das Volumen und die Masse der Abluft pro Zeiteinheit bestimmt werden, ihre Feuchte und Temperatur, sowie der Anteil der trockenen Luft ermittelt werden. Daraus lässt sich dann die Menge des abgeführten Wassers pro Zeiteinheit ermitteln. Es können zudem auch Messungen im Zuluftstrom vorgesehen sein, die gegebenenfalls in die Ermittlung mit einbezogen werden können.

Ebenso können die Mengen an Wassern und Feststoffen -insbesondere Fasern und Füllstoffe- online mittels eines Sensors vor der Aufrollung bestimmt werden.

Jedoch kann es vorteilhaft sein, diese Werte über eine Labormessung zu ermitteln. Die Probennahme an einer aufgewickelten Rolle ist nämlich problem- und gefahrlos möglich, und erfolgt ohnehin meist im Rahmen einer Qualitätskontrolle. Zudem können die Werte zu a) und b) von der fertigen Bahn - zum Beispiel über eine Schnellveraschung -deutlich schneller ermittelt werden, als bei einer Probennahme von der feuchten Bahn vor dem Trockenzylinder. Und schließlich ändern sich diese Werte, speziell die Feststoffmengen nicht so schnell und so stark, so dass die Ermittlung des Trockengehalts vor dem Trockenzylinder nicht sehr stark beeinflusst wird, wenn die Werte zu a) und b) nur in gewissen Intervallen aktualisiert werden.

In weiteren vorteilhaften Ausführungen können zur Bestimmung des Trockengehalts der Faserstoffbahn noch weitere Größen verwendet werden.

Beispielsweise können Sensoren vorgesehen sein, mit denen der Zustand einer Bespannung, z.B. eines Filzes überwacht wird. Dabei kann insbesondere die Filzfeuchte mit in die Bestimmung des Trockengehalts der Faserstoffbahn verwendet werden.

Hinsichtlich der Steuerung oder Regelung wird die Aufgabe gelöst durch ein Verfahren zum Steuern oder Regeln einer Maschine zur Herstellung einer Faserstoffbahn, insbesondere einer Tissuebahn, wobei die Maschine einen Trockenzylinder, insbesondere einen Yankeezylinder umfasst, dem zumindest eine, vorzugsweise zwei Trockenhauben, zugeordnet sind, sowie eine Aufrollung zum Aufwickeln der Faserstoffbahn, und wobei der Trockengehalt der Faserstoffbahn vor dem Trockenzylinder für die Steuerung oder Regelung verwendet wird, dadurch gekennzeichnet, dass der Trockengehalt der Faserstoffbahn mittels eines Verfahrens gemäß einem der Ansprüche 1-3 ermittelt wird.

Vorteilhafte Ausführungen werden in den Unteransprüchen beschrieben.

So kann es insbesondere vorteilhaft sein, wenn der Trockengehalt der Faserstoffbahn während des Betriebs der Maschine online bestimmt wird. Da die direkte Messung des Trockengehalts schwierig ist, kann insbesondere eine indirekte Ermittlung des Trockengehalts erfolgen und für die Steuerung verwendet werden. Üblicherweise werden für diese indirekte Ermittlung Messwerte verwendet, die die dem Betreiber der Maschine ohnehin schon vorliegen. Somit kann die Steuerung oder Regelung besonders kostengünstig und ohne großen Aufwand realisiert werden.

In einer vorteilhaften Ausführung kann vorgesehen sein, dass die Temperatur der zumindest einen Trockenhaube -insbesondere aller Trockenhauben des Trockenzylinders- in Abhängigkeit von dem Trockengehalt der Faserstoffbahn vor dem Trockenzylinder gesteuert oder geregelt wird. So kann beispielsweise bei einem niedrigeren Trockengehalt der Faserstoffbahn vor dem Trockenzylinder die Temperatur der Haubenluft erhöht werden, wodurch die Trocknung verbessert wird.

In einer weiteren vorteilhaften Ausführung kann vorgesehen sein, dass in der Maschine zumindest eine Bespannung, insbesondere ein Filz vorgesehen ist, und die Konditionierung dieser Bespannung in Abhängigkeit von dem Trockengehalt der Faserstoffbahn vor dem Trockenzylinder gesteuert oder geregelt wird.

Häufig wird die Faserstoffbahn auf eine solche Bespannung bzw. einen solchen Filz gestützt zu dem Trockenzylinder geführt. Zur Konditionierung der Bespannung kann - z.B. mittels Hochdruckspritzrohren -Wasser auf die Bespannung aufgebracht werden. Dadurch können unter anderem Verunreinigungen von der Bespannung entfernt werden. Ebenso sind üblicherweise zur Konditionierung auch Absaugeinrichtungen vorgesehen, um überschüssiges Wasser aus der Bespannung zu entfernen.

Je dichter der Filz wird, umso schlechter wird -bei sonst gleichen Bedingungen - der Trockengehalt der Faserstoffbahn vor dem Trockenzylinder sein. Der Trockengehalt dient dann als eine Art Indikator für Zustand des Filzes. Über den ermittelten Trockengehalt könnte dann z.B. die Wassermenge und / oder der Wasserdruck an einem Hochdruckspritzrohr geregelt, und damit die Reinigung des Filzes optimiert werden. Somit kann in dieser Ausführung auf einen Sensor zur Bestimmung des Filzzustandes verzichtet werden.

Vorteilhafterweise können auch zu dieser Regelung noch weitere Werte herangezogen werden, um z.B. sicherzustellen, dass der geänderte Trockengehalt nicht aufgrund eines geänderten Rohstoffs oder geänderter Maschineneinstellungen, wie Vakua an Saugeinrichtungen - entstanden ist.

In einer weiteren vorteilhaften Ausführung kann vorgesehen sein, dass die Dosierung einer Chemikalie, insbesondere eines Ablösemittels in Abhängigkeit von dem Trockengehalt der Faserstoffbahn vor dem Trockenzylinder gesteuert oder geregelt wird. Diese Ablösemittel, die auch als ‚release agents' bekannt sind, werden in der Regel auf den Trockenzylinder gesprüht, um das ablösen oder Abkreppen der Faserstoffbahn von der Zylinderoberfläche zu erleichtern. Diese Chemikalien stellen einen Kostenfaktor dar. Da die Feuchte der Faserstoffbahn einen erheblichen Einfluss auf das Haftverhalten der Bahn am Zylinder hat, kann durch die Kenntnis der Feuchte vor dem Trockenzylindern die Menge an Ablösemittel optimal auf die Bahnverhältnisse angepasst werden. Ein teures Überdosieren des Ablösemittels kann dadurch vermieden werden.

In vielen Ausführungen werden drei Chemikalien in einem bestimmten Mischverhältnis auf den Yankee aufgetragen. Diese sind "Base", "Modifier" und Release agent, wobei das Base die Grundschicht bzw. Schutzschicht für die Yankeeoberfläche bildet, der Modifier die Zeit bestimmt in der das Coating aushärtet und das Release um diese Zeit zu beeinflussen bzw. zu variieren.

Die Chemikalien werden mit Wasser verdünnt auf die Zylinderoberfläche aufgetragen, üblicherweise zwischen Kreppschaber und Pressnip. Ist diese Wassermenge zu hoch, kann dies den Trockengehalt negativ beeinflussen.

Bei allen in dieser Anmeldung beschriebenen Verfahren und Ausführungen kann häufig vorgesehen sein, dass die Faserstoffbahn vor dem Trockenzylinder oder direkt am Trockenzylinder einen Pressnip durchläuft, wobei der Pressnip insbesondere ein Schuhpressnip sein kann.

In vielen Ausführungen läuft die Faserstoffbahn zusammen mit einem Filz durch einen Pressnip, der aus einer Presswalze und einem Yankeezylinder als Gegenwalze gebildet ist. Durch diesen Pressnip wird die Faserstoffbahn dann vom Filz an den Yankeezylinder übergeben. Bei einer derartigen Ausführung ist eine Probennahme dicht vor dem Yankeezylinder besonders schwierig, und zwar sowohl hinsichtlich einer Gefährdung des Personals als auch hinsichtlich einer Beschädigung der Zylinderoberfläche und/oder des Filzes. Daher ist hier ein Verfahren gemäß einem Aspekt der Erfindung besonders vorteilhaft, da hierzu keine Probennahme direkt vor dem Trockenzylinder erfolgen muss.

Zudem ein Computerprogramm zur Durchführung eines erfindungsgemäßen, Verfahrens gemäß einem der Ansprüche 1-8 sowie ein Computerprogrammprodukt mit einem solchen Computerprogramm.

Vorteilhaft kann es sein, wenn ein solches Computerprogramm auf einem Computer ausgeführt wird, und dabei auf Daten aus den Steuer-und Leitsystemen der Maschine zugreifen kann (DCS -maschinennahe Steuerung, QCS - Qualitätsleitsystem). Dort werden häufig bereits viele der Daten erfasst, die zur Durchführung eines Verfahrens gemäß einem Aspekt der Erfindung benötigt werden, z.B. die Abluftmengen, Temperatur und Feuchtigkeit der Haubenabluft, oder Messwerte für die Eigenschaften der Faserstoffbahn an der Aufrollung. Diese Werte können dann von diesen Systemen für die Durchführung des Verfahrens bereitgestellt werden.

Im Folgenden wir die Erfindung anhand einer schematischen, nicht maßstäblichen Figur näher erläutert.

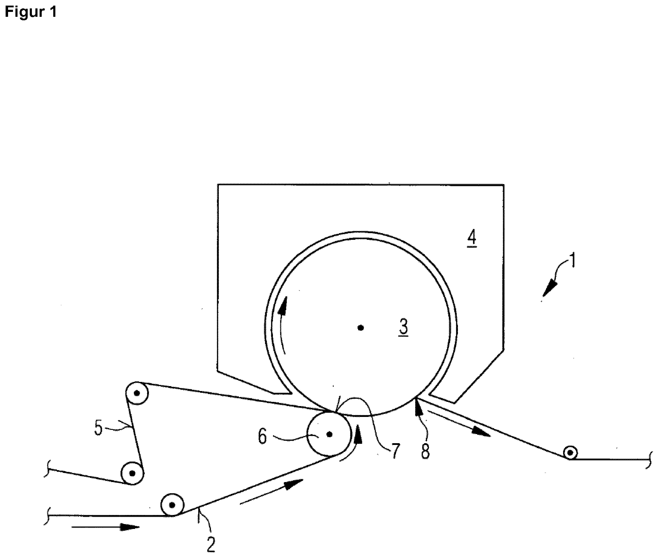

Figur 1 zeigt einen Ausschnitt aus einer Maschine zur Durchführung eines Verfahrens gemäß einem Aspekt der Erfindung.

In Figur 1 ist ein Ausschnitt aus einer Maschine 1 zur Herstellung einer Tissuebahn 2 gezeigt. Die Tissuebahn 2 wird dabei von einem Stoffauflauf kommend, gestützt auf einen Filz 5 transportiert. Es ist ein Pressnip 7 vorgesehen, der aus einer Presswalze 6 sowie einem Yankeezylinder 3 als Gegenwalze gebildet wird. Bei der Presswalze 6 kann es sich um ein Schuhpresswalze 6 handeln. Es kann sich bei dem Pressnip 7 aber auch um einen konventionellen Walzennip 7 handeln. Im Pressnip 7 wird die Faserstoffbahn 2 auf den Yankeezylinder 3 übergeben. Der Filz 5 wird danach ohne die Tissuebahn 3 wieder zurückgeführt. Im Bereich dieses Rücklaufs können noch Einrichtungen zur Filzkonditionierung vorgesehen sein, wie Spritzrohre oder Saugeinrichtungen, die in der Figur 1 nicht dargestellt sind. Ebenso können Messeinrichtungen für den Filzzustand vorgesehen sein.

Der Yankeezylinder 3 ist üblicherweise dampfbeheizt. In der in Figur 1 gezeigten Maschine 1 ist dem Yankeezylinder 3 eine Trockenhaube 4 zugeordnet. In anderen vorteilhaften Ausführungen können auch zwei Trockenhauben 4 vorgesehen sein. Diese sind dann in der Regel direkt hintereinander angeordnet und werden mit Nassteil und Trockenteil bezeichnet. Nach dem Verlassen der Trockenhaube 4 wird die Materialbahn 2 vom Yankeezylinder am Ablösepunkt 8 abgelöst oder abgekreppt, und in Richtung der Aufrollung weitergeführt, welche nicht dargestellt ist. Zur Erleichterung der Abnahme der Tissuebahn 2 vom Yankeezylinder 3, kann auf die Oberfläche des Yankeezylinders 3 ein Trennmittel aufgebracht werden. Das Aufbringen kann beispielsweise durch Sprühauftrag erfolgen, und geschieht zweckmäßigerweise in dem Bereich des Yankeezylinders 3, der nicht von der Tissuebahn 2 bedeckt ist, also zwischen dem Ablösepunkt 8 und dem Pressnip.

Die Trockenhaube 4 erstreckt sich bei der Maschine in Figur 1 über einen sehr großen Teil der Oberfläche des Yankeezylinders 3. Durch sie wird heiße Luft auf die Faserstoffbahn 2 geführt. Generell kann die Lufttemperatur in Trockenhauben 4, die für Aspekte dieser Erfindung geeignet sind, vorteilhafterweise zwischen 150°C und 500° C, insbesondere zwischen 250°C und 400°C liegen. Bei dampfbeheizten Trockenhauben 4 überschreitet die Temperatur selten 200°C. Für höhere Temperaturen kommen üblicherweise gasbeheizte Trockenhauben 4 zum Einsatz. Durch die Trocknung der Tissuebahn 2 tritt dabei Feuchtigkeit aus der Tissuebahn 2. aus und gelangt in die Trockenhaube 4 und reichert sich darin an. Aus diesem Grund wird oft meist ein Teil dieser feuchten Haubenluft als Abluft abgeführt, und trockene Luft der Trockenhaube 4 als Zuluft zugeführt. Durch Messungen im Abluftstrom - und gegebenenfalls im Zuluftstrom - kann online, während des laufenden Betriebs der Maschine 1 sehr zuverlässig die Menge des pro Zeiteinheit mit der Abluft mitgeführten Wassers ermittelt werden, welche in sehr guter Näherung der Menge des in dieser Zeit aus der Tissuebahn 2 durch Trocknung entfernten Wassers entspricht. Die Kenntnis dieser Größe ist eine wichtige Größe für die Durchführung des Verfahrens gemäß einigen Aspekten der Erfindung. Zusammen mit der Kenntnis der Wassermenge und der Feststoffmenge (Fasern, Füllstoffe etc.) der Tissuebahn 2 an der Aufrollung, bzw. einer ausreichend guten Näherung dafür, kann der Trockengehalt der Faserstoffbahn nach dem Pressnip 7, bzw. bei Eintritt in die Trockenhaube 4 in sehr guter Näherung bestimmt werden. (Diese beiden Punkte liegen bei der Ausführung gemäß Figur 1 sehr nahe benachbart, wodurch unter anderem auch eine Probennahme der Tissuebahn 2 in diesem Bereich sehr schwierig und gefährlich ist.).

Der online verfügbare Trockengehalt vor dem Yankeezylinder 3 kann für verschiedenen Steuerungen oder Regelungen verwendet werden.

So kann beispielsweise die Temperatur der Haubenluft der zumindest einen Trockenhaube 4 in Abhängigkeit von dem Trockengehalt der Faserstoffbahn 2 vor dem Trockenzylinder 3 gesteuert oder geregelt werden. Diese kann vorteilhafterweise im Bereich zwischen 205°C und 360°C liegen.

Alternativ oder zusätzlich kann die Dosierung einer Chemikalie, insbesondere eines Ablösemittels in Abhängigkeit von dem Trockengehalt der Faserstoffbahn 2 vor dem Trockenzylinder 3 gesteuert oder geregelt werden.

Alternativ oder zusätzlich kann auch die Konditionierung einer Bespannung 5 in Abhängigkeit von dem Trockengehalt der Faserstoffbahn 2 vor dem Trockenzylinder 3 gesteuert oder geregelt werden.

Diese Beispiele für Regelungen stellen vorteilhafte Ausführungen dar. Die Erfindung ist jedoch nicht auf die beschriebenen Regelungen beschränkt.

**Patentansprüche**

1. Verfahren zur Bestimmung des Trockengehalts einer Faserstoffbahn (2), insbesondere eine Tissuebahn (2), während der Herstellung der Faserstoffbahn (2) in einer Maschine (1), wobei die Maschine (1) einen Trockenzylinder (3), insbesondere einen Yankeezylinder (3) umfasst, dem zumindest eine, vorzugsweise zwei Trockenhauben (4), zugeordnet sind, sowie eine Aufrollung zum Aufwickeln der Faserstoffbahn (2) **dadurch gekennzeichnet, dass** die Bestimmung des Trockengehalts der Faserstoffbahn (2) vor dem Trockenzylinder (3) auf Basis von Messwerten erfolgt, die die folgenden Größen Beschreiben:

   a) Feststoffmenge der Faserstoffbahn (2) an der Aufrollung
   b) Wassermenge der Faserstoffbahn (2) an der Aufrollung
   c) Wassermenge, die in der oder den Trockenhauben verdampft wird.

2. Verfahren gemäß Anspruch 1, **dadurch gekennzeichnet, dass** die Maschine (1) Messvorrichtungen umfasst, um zumindest eine, vorzugsweise alle Größen zu a) b) und/oder c) während des laufenden Betriebs der Maschine (1) online zu erfassen.

3. Verfahren gemäß einem der vorherigen Ansprüche, **dadurch gekennzeichnet, dass** zur Bestimmung des Trockengehalts der Faserstoffbahn (2) noch weitere Größen verwendet werden.

**4.** Verfahren zum Steuern oder Regeln einer Maschine (1) zur Herstellung einer Faserstoffbahn (2), insbesondere einer Tissuebahn (2), wobei die Maschine (1) einen Trockenzylinder (3), insbesondere einen Yankeezylinder (3) umfasst, dem zumindest eine, vorzugsweise zwei Trockenhauben (4), zugeordnet sind, sowie eine Aufrollung zum Aufwickeln der Faserstoffbahn (2), und wobei der Trockengehalt der Faserstoffbahn (2) vor dem Trockenzylinder (3) für die Steuerung oder Regelung verwendet wird, **dadurch gekennzeichnet, dass** der Trockengehalt der Faserstoffbahn (2) mittels eines Verfahrens gemäß einem der Ansprüche 1 - 3 ermittelt wird.

**5.** Verfahren gemäß Anspruch 4, **dadurch gekennzeichnet, dass** der Trockengehalt der Faserstoffbahn (2) während des Betriebs der Maschine (1) online bestimmt wird.

**6.** Verfahren nach Anspruch 4 oder 5 **dadurch gekennzeichnet, dass** die Temperatur der zumindest einen Trockenhaube (4) in Abhängigkeit von dem Trockengehalt der Faserstoffbahn (2) vor dem Trockenzylinder (3) gesteuert oder geregelt wird.

**7.** Verfahren nach einem der Ansprüche 4 bis 6, **dadurch gekennzeichnet, dass** in der Maschine zumindest eine Bespannung (5), insbesondere ein Filz (5) vorgesehen ist, und die Konditionierung dieser Bespannung (5) in Abhängigkeit von dem Trockengehalt der Faserstoffbahn (2) vor dem Trockenzylinder (3) gesteuert oder geregelt wird.

**8.** Verfahren nach einem der Ansprüche 4 bis 7, **dadurch gekennzeichnet, dass** die Dosierung einer Chemikalie, insbesondere eines Ablösemittels in Abhängigkeit von dem Trockengehalt der Faserstoffbahn (2) vor dem Trockenzylinder (3) gesteuert oder geregelt wird.

**9.** Verfahren gemäß einem der vorherigen Ansprüche, **dadurch gekennzeichnet, dass** die Faserstoffbahn (2) vor dem Trockenzylinder (3) oder direkt am Trockenzylinder (3) einen Pressnip (7) durchläuft, wobei der Pressnip (7) insbesondere ein Schuhpressnip (7) ist.

**10.** Computerprogramm zur Durchführung eines Verfahrens gemäß einem der vorherigen Ansprüche.

**11.** Computerprogrammprodukt mit einem Computerprogramm gemäß Anspruch 10.


**Claims**

**1.** Method for determining the dryness of a fibrous web (2), in particular a tissue web (2), during the production of the fibrous web (2) in a machine (1), wherein the machine (1) comprises a drying cylinder (3), in particular a Yankee cylinder (3), to which at least one, preferably two, dryer hoods (4) are assigned, and a reel-up for winding up the fibrous web (2), **characterized in that** the determination of the dryness of the fibrous web (2) is carried out before the drying cylinder (3) on the basis of measured values which describe the following variables:

   a) the amount of solids in the fibrous web (2) at the reel-up,
   b) the amount of water in the fibrous web (2) at the reel-up,
   c) the amount of water which is evaporated in the dryer hood or hoods.

**2.** Method according to Claim 1, **characterized in that** the machine (1) comprises measuring devices in order to acquire at least one, preferably all, the variables relating to a), b) and/or c) online during running operation of the machine (1).

**3.** Method according to one of the preceding claims, **characterized in that** still further variables are used to determine the dryness of the fibrous web (2).

**4.** Method for controlling or regulating a machine (1) for producing a fibrous web (2), in particular a tissue web (2), wherein the machine (1) comprises a drying cylinder (3), in particular a Yankee cylinder (3), to which at least one, preferably two, dryer hoods (4) are assigned, and a reel-up for winding up the fibrous web (2), and wherein the dryness of the fibrous web (2) before the drying cylinder (3) is used for the control or regulation, **characterized in that the** dryness of the fibrous web (2) is determined by means of a method according to one of Claims 1 - 3.

**5.** Method according to Claim 4, **characterized in that** the dryness of the fibrous web (2) is determined online during the operation of the machine (1).

6. Method according to Claim 4 or 5, **characterized in that** the temperature of the at least one dryer hood (4) is controlled or regulated as a function of the dryness of the fibrous web (2) before the drying cylinder (3).

7. Method according to one of claims 4 to 6, **characterized in that** at least one fabric (5), in particular a felt (5), is provided in the machine, and the conditioning of this fabric (5) is controlled or regulated as a function of the dryness of the fibrous web (2) before the drying cylinder (3).

8. Method according to one of Claims 4 to 7, **characterized in that** the dosing of a chemical, in particular a release agent, is controlled or regulated as a function of the dryness of the fibrous web (2) before the drying cylinder (3).

9. Method according to one of the preceding claims, **characterized in that** the fibrous web (2) passes through a press nip (7) before the drying cylinder (3) or directly on the drying cylinder (3), wherein the press nip (7) is in particular a shoe press nip (7).

10. Computer program for carrying out a method according to one of the preceding claims.

11. Computer program product having a computer program according to Claim 10.


**Revendications**

1. Procédé de détermination de la teneur en matière sèche d'une bande de matière fibreuse (2), en particulier d'une bande de papier mousseline (2), pendant la fabrication de la bande de matière fibreuse (2) dans une machine (1), la machine (1) comportant un cylindre de séchage (3), en particulier un cylindre Yankee (3), auquel sont associées au moins une, de préférence deux hottes de séchage (4), ainsi qu'un dispositif d'enroulement pour l'embobinage de la bande de matière fibreuse (2), **caractérisé en ce que** la détermination de la teneur en matière sèche de la bande de matière fibreuse (2) a lieu avant le cylindre de séchage (3) sur la base de valeurs mesurées qui décrivent les grandeurs suivantes :

   a) la quantité de matière solide de la bande de matière fibreuse (2) au niveau du dispositif d'enroulement,
   b) la quantité d'eau de la bande de matière fibreuse (2) au niveau du dispositif d'enroulement,
   c) la quantité d'eau qui est évaporée dans la ou les hottes de séchage.

2. Procédé selon la revendication 1, **caractérisé en ce que** la machine (1) comporte des dispositifs de mesure, afin de déterminer en ligne au moins une, de préférence toutes les grandeurs selon a), b) et/ou c) pendant l'exploitation courante de la machine (1).

3. Procédé selon l'une quelconque des revendications précédentes, **caractérisé en ce que** des grandeurs supplémentaires sont encore utilisées pour la détermination de la teneur en matière sèche de la bande de matière fibreuse (2).

4. Procédé de commande ou de réglage d'une machine (1) pour la fabrication d'une bande de matière fibreuse (2), en particulier d'une bande de papier mousseline (2), la machine (1) comportant un cylindre de séchage (3), en particulier un cylindre Yankee (3), auquel sont associées au moins une, de préférence deux hottes de séchage (4), ainsi qu'un dispositif d'enroulement pour l'embobinage de la bande de matière fibreuse (2), et la teneur en matière sèche de la bande de matière fibreuse (2) avant le cylindre de séchage (3) étant utilisée pour la commande ou le réglage, **caractérisé en ce que** la teneur en matière sèche de la bande de matière fibreuse (2) est déterminée au moyen d'un procédé selon l'une quelconque des revendications 1 à 3.

5. Procédé selon la revendication 4, **caractérisé en ce que** la teneur en matière sèche de la bande de matière fibreuse (2) est déterminée en ligne pendant l'exploitation de la machine (1).

6. Procédé selon la revendication 4 ou 5, **caractérisé en ce que** la température de l'au moins une hotte de séchage (4) est commandée ou réglée en fonction de la teneur en matière sèche de la bande de matière fibreuse (2) avant le cylindre de séchage (3).

7. Procédé selon l'une quelconque des revendications 4 à 6, **caractérisé en ce qu'**au moins une toile (5), en particulier un feutre (5), est prévue dans la machine, et le conditionnement de cette toile (5) est commandé ou réglé en fonction de la teneur en matière sèche de la bande de matière fibreuse (2) avant le cylindre de séchage (3).

8. Procédé selon l'une quelconque des revendications 4 à 7, **caractérisé en ce que** l'ajout dosé d'un produit chimique, en particulier d'un agent de décollement, est commandé ou réglé en fonction de la teneur en matière sèche de la bande de matière fibreuse (2) avant le cylindre de séchage (3).

9. Procédé selon l'une quelconque des revendications précédentes, **caractérisé en ce que** la bande de matière fibreuse (2) traverse une ligne de contact de pressage (7) avant le cylindre de séchage (3) ou directement au niveau du cylindre de séchage (3), la ligne de contact de pressage (7) étant en particulier une ligne de contact de pressage à sabot (7).

10. Programme informatique pour la réalisation d'un procédé selon l'une quelconque des revendications précédentes.

11. Produit de programme informatique comprenant un programme informatique selon la revendication 10.

**Figur 1**

**IN DER BESCHREIBUNG AUFGEFÜHRTE DOKUMENTE**

**In der Beschreibung aufgeführte Patentdokumente**

- DE 102012203035
- US 9284686 B1